(19) **Europäisches Patentamt / European Patent Office / Office européen des brevets**

(11) **EP 3 557 253 A1**

(12) # EUROPEAN PATENT APPLICATION
published in accordance with Art. 153(4) EPC

(43) Date of publication:
**23.10.2019 Bulletin 2019/43**

(21) Application number: **17882015.5**

(22) Date of filing: **12.09.2017**

(51) Int Cl.:
*G01N 33/543* (2006.01)   *G01N 21/49* (2006.01)
*G01N 21/75* (2006.01)   *G01N 21/82* (2006.01)

(86) International application number:
**PCT/JP2017/032822**

(87) International publication number:
**WO 2018/110006 (21.06.2018 Gazette 2018/25)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD**

(30) Priority: **15.12.2016 JP 2016243796**

(71) Applicant: **Horiba, Ltd.**
**Kyoto-shi, Kyoto 601-8510 (JP)**

(72) Inventors:
• **IGUSHI, Tatsuo**
  **Kyoto-shi**
  **Kyoto 601-8510 (JP)**
• **MAEDA, Takuto**
  **Kyoto-shi**
  **Kyoto 601-8510 (JP)**

(74) Representative: **Isarpatent**
**Patent- und Rechtsanwälte Behnisch Barth Charles**
**Hassa Peckmann & Partner mbB**
**Friedrichstrasse 31**
**80801 München (DE)**

(54) **METHOD FOR ASSESSING APPROPRIATENESS OF TEST SUBSTANCE CONCENTRATION IN CONCENTRATION MEASUREMENT USING IMMUNOAGGLUTINATION REACTION, AND SAMPLE ANALYSIS DEVICE HAVING PROCESSING UNIT FOR SAME**

(57)   A method and a sample analysis apparatus having a processing part for practicing the method, the method including a step of, with respect to an immunoagglutination reaction that occurred in the reaction solution between the test substance and a binding partner thereof, determining an index value relating to a difference between a changing speed of a scattered light intensity in a first period of the reaction and a changing speed of a scattered light intensity in a second period of the reaction, based on a scattered light intensity of the reaction solution in the first period and a scattered light intensity of the reaction solution in the second period, and a step of determining whether the concentration of the test substance is within a proper range by comparing the index value and a predetermined threshold value.

**Description**

[Technical Field]

The present invention generally relates to the technical field of a concentration measurement method using an immunoagglutination reaction. More particularly, it relates to a method for determining whether a concentration of a test substance is within an appropriate range for concentration measurement using an immunoagglutination reaction, and a sample analysis apparatus having a processing part therefor.

[Background Art]

In concentration measurement methods using an immunoagglutination reaction, absorbance measurement and scattered light measurement are used as optical measurement methods by an automatic analysis apparatus using an appropriate measurement reagent (patent document 1).

In these optical measurements, a test substance is quantified using an analytical curve showing the relationship between the changing speed of turbidity and the specimen concentration after a particular time from the start of the reaction and based on the measured changing speed of the turbidity. In the analytical curve, the slope becomes smaller as the concentration of the test substance becomes higher, and a region with a reverse gradient (prozone region) is developed in higher concentration regions.

In the prozone region, two solutions are generated from an analytical curve, and quantitative determination cannot be performed. Therefore, it is important in a measurement using an automatic analysis apparatus to determine whether the test object contains a high concentration substance in the prozone region, and determine whether or not a test substance is measured in the prozone region.

Conventionally, the value of absorbance in initial reaction, time before stabilization of absorbance, changing speed of absorbance in initial stage of reaction and the like are used for the determination of the prozone region.

[Document List]

[Patent document]

patent document 1: WO 2014/192963 A1

[SUMMARY OF THE INVENTION]

[Problems to be Solved by the Invention]

In the determination of the prozone region in the Prior Art described above, the measurement time is set sufficiently long for the reaction to reach the equilibrium, and the initial absorbance in a predetermined time, change amount of absorbance up to that time and the like are utilized. However, to complete the measurement in a shorter time, a method capable of determining prozone in the initial stage of the reaction is desirable.

In addition, a robust determination method less susceptible to an influence of variation or dispersion in the measurement results due to reagent variation and the like is desired.

The present invention has been made in view of the aforementioned problem in the pertinent technical field, and aims to provide a method for determining in a short time whether a concentration of a test substance is within an appropriate range for concentration measurement using an immunoagglutination reaction, which method being less susceptible to an influence of measurement variation, and a sample analysis apparatus having a processing part therefor.

[Means of Solving the Problems]

The present inventors have conducted intensive studies of the aforementioned problems and found that the appropriateness of the concentration of a test substance can be determined even in a short time when the reaction has not reached equilibrium by determining the ratio between a turbidity changing speed in a first period and a turbidity changing speed in a second period of an immunoagglutination reaction by measuring absorbance (transmitted light), and comparing the ratio with a predetermined threshold value. However, when the above-mentioned ratio obtained by the absorbance measurement is used, the difference between the measured value of the ratio and the threshold value is small at test substance concentrations close to the prozone region, and thus the values are problematically susceptible to the variation of the measurement results including the reagent variation.

Therefore, the present inventors considered further improvement and envisaged a methodology using an index value relating to the changes between the changing speed of the scattered light intensity in the first period of the reaction and the changing speed of the scattered light intensity in the second period instead of the above-mentioned ratio determined by measuring the absorbance. They have found that an index value obtained by measuring the scattered light intensity can increase the degree of increase in the index value which is caused by an increase in the concentration of the test substance, as compared to when a transmitted light is used, and that the index value reaches a certain value or above in a high concentration range near the prozone region. Thus, it was found that the difference between the threshold value for prozone determination and the index value to be measured can be increased even when the concentration of the test substance is in the vicinity of the prozone region, thereby making measurement variation less influential. The present inventors have conducted further studies based on these findings and completed the present invention.

That is, the present invention provides the following.

[1] A method for determining whether a concentration of a test substance in a reaction solution is within a range proper for concentration measurement using an immunoagglutination reaction, the method comprising:

a step of, with respect to an immunoagglutination reaction that occurred in the reaction solution between the test substance and a binding partner thereof, determining an index value relating to a difference between a changing speed of a scattered light intensity in a first period of the reaction and a changing speed of a scattered light intensity in a second period of the reaction, based on a scattered light intensity of the reaction solution in the first period and a scattered light intensity of the reaction solution in the second period; and
a step of determining whether the concentration of the test substance is within a proper range by comparing the index value and a predetermined threshold value.

[2] The method according to the above-mentioned [1], wherein the index value is a ratio of the changing speed of the scattered light intensity in the first period and the changing speed of the scattered light intensity in the second period.

[3] The method according to the above-mentioned [1] or [2], wherein a ratio of the measurement value of the scattered light intensity and the measurement value of the transmitted light intensity is used as the aforementioned scattered light intensity.

[4] The method according to any one of the above-mentioned [1] to [3], wherein the reaction solution is derived from a wet biological sample comprising the test substance.

[5] The method according to the above-mentioned [4], wherein the wet biological sample is selected from the group consisting of blood, saliva, urine, sweat, lymph fluid, and expectoration.

[6] The method according to any one of the above-mentioned [1] to [5], wherein the test substance is selected from the group consisting of protein, peptide, lipid, nucleic acid, polysaccharides, saccharides, and antibody.

[7] The method according to any one of the above-mentioned [1] to [6], wherein the reaction solution is prepared by mixing a specimen sample containing the test substance and a suspension containing insoluble carrier particles carrying the binding partner.

[8] The method according to the above-mentioned [7], wherein the insoluble carrier particle comprises one or more selected from the group consisting of latex, metal colloid, silica, carbon and magnetic material particles.

[9] The method according to any one of the above-mentioned [1] to [8], wherein the concentration in the proper range is a concentration which is neither a concentration causing a prozone phenomenon nor a concentration around same.

[10] The method according to any one of the above-mentioned [1] to [9], further comprising a step of measuring the concentration of the test substance, and determining that the concentration of the test substance is not within the proper range when the measured concentration is lower than a predetermined threshold value concentration.

[11] A sample analysis apparatus comprising:

a scattered light intensity measuring part for measuring intensity of a scattered light;
an index value determining part for, with respect to an immunoagglutination reaction that occurred in the reaction solution between the test substance and a binding partner thereof, determining an index value relating to a difference between a changing speed of a scattered light intensity in a first period of the reaction and a changing speed of a scattered light intensity in a second period of the reaction, based on a scattered light intensity of the reaction solution in the first period and a scattered light intensity of the reaction solution in the second period; and
a first determining part for determining whether the concentration of the test substance is within a range proper for concentration measurement using an immunoagglutination reaction, by comparing the index value and a predetermined threshold value.

[12] The sample analysis apparatus according to the above-mentioned [11], wherein the index value is a ratio of the changing speed of the scattered light intensity in the first period and the changing speed of the scattered light intensity in the second period.

[13] The sample analysis apparatus according to the above-mentioned [11] or [12], further comprising a transmitted light intensity measuring part for measuring a transmitted light intensity, wherein a ratio of the measurement value of the scattered light intensity and the measurement value of the transmitted light intensity is used as the aforementioned scattered light intensity.

[14] The sample analysis apparatus according to any one of the above-mentioned [11] to [13], which is used for analyzing a wet biological sample comprising the test substance.

[15] The sample analysis apparatus according to the above-mentioned [14], wherein the wet biological sample is selected from the group consisting of blood, saliva, urine, sweat, lymph fluid, and expectoration.

[16] The sample analysis apparatus according to any one of the above-mentioned [11] to [15], wherein the first determining part determines whether the concentration of the test substance is a concentration which is neither a concentration causing a prozone phenomenon nor a concentration in the vicinity thereof.

[17] The sample analysis apparatus according to any one of the above-mentioned [11] to [16], further comprising a concentration measuring part for measuring the concentration of the test substance.

[18] The sample analysis apparatus according to the above-mentioned [17], further comprising a second determining part for determining that the concentration of the test substance is not within the proper range when the measured concentration of the test substance is lower than a predetermined threshold value concentration.

[Effect of the Invention]

According to the present invention, there are provided a method for determining in a short time whether a concentration of a test substance is within an appropriate range for concentration measurement using an immunoagglutination reaction, which method being less susceptible to an influence of measurement variation, and a sample analysis apparatus having a processing part therefor.

[Brief Description of the Drawings]

Fig. 1 is a block diagram showing the outline of the constitution of one embodiment of the sample analysis apparatus of the present invention.

Fig. 2 is a block diagram showing the outline of the constitution example of an optical system that the sample analysis apparatus of the present invention may have.

Fig. 3 is a block diagram showing the outline of the constitution of other embodiment of the sample analysis apparatus of the present invention.

Fig. 4 is a graph showing changes in the indicated measurement value when a serum containing a known concentration of CRP is measured.

Fig. 5 is a graph showing changes in the serum CRP concentration for the deflection value when scattered light and absorbance are used.

[Description of Embodiments]

The determination method of the present invention and the sample analysis apparatus having a processing part therefor of the present invention are explained in detail by showing illustrative embodiments thereof.

(Determination method)

The present invention provides a method for determining whether a concentration of a test substance in a reaction solution is within a range proper for measurement of concentration using an immunoagglutination reaction.

The determination method of the present invention includes:

a step of, with respect to an immunoagglutination reaction between the test substance and a binding partner thereof that occurred in the reaction solution, determining an index value relating to a difference between changing speed of the scattered light intensity in the first period and the changing speed of the scattered light intensity in the second period based on the scattered light intensity of the reaction solution in a first period of the reaction and the scattered light intensity of the reaction solution in a second period of the reaction; and

a step of determining whether the concentration of the test substance is within a proper range by comparing the index value and a predetermined threshold value.

In the present specification, the "immunoagglutination reaction" refers to a reaction in which aggregation occurs by a reaction between an antigen and an antibody. In the present invention, it particularly refers to a reaction in which aggregation of particles present in the reaction solution occurs in response to an antigen-antibody reaction between a test substance and its binding partner. The aggregation may increase the turbidity of the reaction solution. The concentration of a test substance is measured by quantifying aggregation of particles that are generated by an immunoagglutination reaction. Generally, it is known that, in a concentration measurement method using an immunoagglutination reaction, when the amount of an antigen or antibody in an antigen-antibody reaction is in excess, decreased signal values are measured, as a result of which a phenomenon occurs in which a test substance concentration lower than the concentration that should be achieved normally is obtained or identification of the concentration of the test substance to only one from the signal value alone is not attainable. This phenomenon is called a "prozone phenomenon" or "Hook effect". In one embodiment, the determination method of the present invention determines whether a prozone phenomenon has occurred in a concentration measurement a test substance using an immunoagglutination reaction. Generally, measurement results may vary due to variation in the amounts of reagents and the like. Thus, a concentration range in the vicinity of the concentration region where the prozone phenomenon occurs (to be also referred to as prozone region in the present specification) also may not be appropriate for the concentration measurement. In a preferable embodiment, therefore, the determination method of the present invention is a method for determining that the concentration of a test substance is not a concentration within the prozone region or a region in the vicinity thereof (in other words, not a concentration at which development of the prozone phenomenon is suspected).

In addition, the determination method of the present invention may further contain a step of measuring the concentration of the test substance, and determining that the concentration of the test substance is not within the proper range when the measured concentration is lower than a predetermined threshold value concentration. This step is preferably performed before the step of determining the "index value" in the present invention. This is because the measurement reproducibility is generally not good in the low concentration region and numerical value of the index is not stable because the turbidity change is small at the beginning after the start of the reaction.

The determination method of the present invention can be performed together with the measurement of the concentration of a test substance by using an immunoagglutination reaction. Using the determination method of the present invention, it can be determined whether the measurement of a test substance within a proper concentration range was performed. A method for obtaining the concentration measurement value is not particularly limited. For example, transmitted light (absorbance), scattered light, or transmitted light and scattered light are measured, and the concentration can be determined on the basis of the obtained signal value and analytical curve.

In the present specification, "the reaction solution" is not particularly limited as long as an immunoagglutination reaction proceeds by the co-presence of a test substance, a binding partner thereof and particles in the solution. The reaction solution can be prepared by, for example, mixing a sample containing a test substance and (i) a solution containing the binding partner and particles, or (ii) a solution containing the binding partner and a solution containing the particles.

The sample to be the analysis target in the present invention is not particularly limited and is generally a wet biological sample. Examples of the wet biological sample include body fluids such as blood (whole blood, serum, plasma), saliva, urine, sweat, lymph fluid, expectoration and the like. The "sample containing a test substance" also includes not only the above-mentioned sample itself but also samples that have been prepared for various analysis purposes (e.g., mixing with buffer etc., dilution and the like). For dilution, buffer at pH 5 - 11, preferably pH 6 - 10, specifically, phosphate buffer, glycine buffer, tris buffer, borate buffer, citrate buffer and the like can be used.

In the present specification, "a test substance" is not particularly limited as long as the concentration can be measured using an immunoagglutination reaction. The test substance may be, for example, an antigen such as protein, peptide, lipid, nucleic acid, polysaccharides,'saccharides or antibody and the like. Examples of the antigen include various antigens, receptor, hormone and hormone-like substance, drug, enzyme and the like. Specific examples thereof include C-reactive protein (CRP), insulin, human fibrinogen, micro albumin, type IV collagen, mycoplasma antigen, HBs antigen, hemoglobin A1c and the like. Examples of the antibody include autoantibody related to various diseases, antibodies against various toxins, pathogenic bacteria and the like, and the like. Specific examples thereof include autoantibodies such as rheumatoid factor, anti-nuclear antibody, anti-CCP antibody, anti-DNA antibody, anti-ENA antibody, anti-intrinsic factor antibody, anti-phospholipid antibody and the like, anti-syphilis antigen antibody, anti-treponema pallidum antibody, anti-HBs antibody, anti-HBc antibody, anti-HBe antibody, anti-insulin antibody, anti-CRP antibody, anti-phospholipid antibody and the like.

The binding partner with the test substance is not particularly limited as long as it can cause an antigen-antibody reaction with the test substance and can induce aggregation of particles by the reaction. It may require or desire additional components (e.g., secondary antibody capable of specifically binding to a binding partner that is the primary antibody) to induce aggregation of particles. The binding partner may be, for example, an antigen such as protein, peptide, lipid, nucleic acid, polysaccharides and saccharides, or an antibody, or the like.

The binding partner may be carried on the particles at the time point of preparation of the reaction solution or may not be carried on the particles at this time point (examples of the latter case include measurement of hemoglobin A1c and the like). The amount of the binding partner to be carried on the particles and a quantitative ratio of the binding partner and the particles vary depending on the concrete substances to be used and the like and are not particularly limited.

The particles are not particularly limited as long as it can induce aggregation by an antigen-antibody reaction between a test substance and a binding partner thereof. The particle is preferably an insoluble carrier particle. As the insoluble carrier particle, a known carrier particle can be used. Examples of the material of the carrier include synthetic polymer powders such as polystyrene, styrene-methacrylic acid copolymer, styrene-glycidyl (meth)acrylate copolymer, styrenestyrene sulfonate copolymer, methacrylic acid polymer, acrylic acid polymer, acrylonitrile-butadiene-styrene copolymer, vinyl chloride-acrylate copolymer, polyvinyl acetate acrylate and the like, metal colloid (gold, titanium, nickel etc.), silica, carbon, magnetic material particles and the like. Latexes in which various polymer fine particles are stably suspended in water or an aqueous medium are frequently used in the art, and can also be recited as preferable carrier particles in the present invention.

The particle size of the insoluble carrier particles is not particularly limited and varies depending on the measurement method to be used in the present invention, material of particles, and the like. It is generally 0.01 - 1.0 $\mu$m, preferably about 0.1 - 0.7 $\mu$m. The concentration of the carrier particles in the solution may be a concentration generally used for an immunoagglutination reaction, and is generally preferably 1 - 20 wt%.

As mentioned above, the binding partner may be carried on particles (preferably insoluble carrier particles) at the time point of preparing the reaction solution. A method for carrying a binding partner on insoluble carrier particles is not particularly limited, and conventionally-known methods such as a physical adsorption method and a method using a chemical binding by a covalent bond and the like can be used. After carrying, known treatments such as a blocking treatment using BSA (bovine serum albumin) and the like, and the like may be performed as appropriate.

The solution containing a binding partner and/or particles may be based on water or an aqueous medium. The solution may further contain additional components such as stabilizer, aggregation promoter and the like. Alternatively, these additional components may be added to a sample solution containing a test substance, or separately added to a reaction solution in which an immunoagglutination reaction occurs. Examples of the stabilizer include the aforementioned buffers, synthetic or natural polymers such as polyethylene glycol, polysaccharides and the like, surfactants and the like. Examples of the aggregation promoter include synthetic or natural polymers such as polyethylene glycol, polyvinylpyrrolidone, carboxymethylcellulose, dextran, pullulan, phospholipid polymer and the like. In addition, surfactant, synthetic or natural polymer, organic or inorganic reagent, and the like may be added as appropriate to any solution or reaction solution before mixing.

By mixing the aforementioned sample solution containing a test substance and one or more solutions containing a binding partner and/or particles (optionally, further solution containing addition component), an immunoagglutination reaction can be started in the mixture (the reaction mixture).

After the start of the immunoagglutination reaction, an index value relating to a difference between changing speed of the scattered light intensity in the first period and the changing speed of the scattered light intensity in the second period is determined based on the scattered light intensity of the reaction solution in a first period of the reaction and the scattered light intensity of the reaction solution in a second period of the reaction.

As the first period and the second period, any period may be selected as long as it does not markedly impair the reliability of the index value determined. The first period and the second period may be partly overlapped. Generally, stable signals may not be achieved immediately after the start of the reaction (e.g., up to 5 seconds from the start of reaction) since the reaction solution is not yet stirred sufficiently. Thus, it is preferable to not use this period as the first period or the second period. In addition, to enable determination in a short time, the first period and the second period are, for example, each a period within 5 minutes, preferably within 3 minutes, more preferably within 2 minutes, further preferably within 1 minute, from the start of the reaction, although it varies depending on the specific reaction system.

In one embodiment, one of the first period and the second period is a period in which the turbidity changes rapidly after the start of the reaction (former half of reaction) and the other is a period in which the turbidity has become gradual after the period in which the turbidity changes rapidly (latter half of reaction). More particularly, for example, the former half of the reaction may include a time point when the turbidity is 20% of the turbidity when the reaction is in equilibrium, and the latter half of the reaction may include a time point when the turbidity is 70% of the turbidity when the reaction is in equilibrium. In fact, these periods may be determined by optionally setting the measurement time, and appropriately defining the former half and the latter half from the measurement period. While it varies depending on the target reaction system, specifically, for example, in the case of one time measurement, the former half of the reaction may be 11 to 30 seconds from the start of the reaction and the latter half of the reaction may be 41 to 60 seconds from the start of the reaction.

The scattered light intensity used for determining the above-mentioned index value may be the scattered light intensity of the reaction solution measured at two or more time points in the first period and the scattered light intensity of the reaction solution measured at two or more time points in the second period. In this case, the length in time of the first period and the second period (i.e., maximum time difference between the two or more time points) is not particularly limited. From the aspect of enhanced reliability of the changing speed of the scattered light intensity in each period, it is generally 10 - 25 seconds, preferably 15 - 20 seconds.

The changing speed of the scattered light intensity in the first period or the second period is not particularly limited as long as it indicates the degree of changing speed of the scattered light intensity within the period. It may be a changing speed at a particular time point in each period or a whole changing speed in each period. Specifically, the changing speed can be determined by, for example, (a) determining the change amount of the scattered light intensity per unit time at each time point by measuring the scattered light intensity at equal intervals (e.g., every second) within the period, and averaging same over the period, or (b) measuring the scattered light intensity at any two time points within the period, and dividing the change amount between the measured values by the time length between the two points or the like. Alternatively, a graph about the time change of the scattered light intensity is created from the measured values of the scattered light intensity at multiple time points, and the changing speed may be determined from the slope of the tangent of the curve in the graph.

In the present invention, the "scattered light intensity" is not particularly limited as long as it quantitatively shows the intensity of a scattered light from a reaction solution to be the measurement target. In a preferable embodiment, as the intensity of scattered light at a given time point t, a ratio of the measurement value of the scattered light intensity at time point t and the measurement value of the transmitted light intensity can be used, as shown in the following formula:

$$\text{Scattered light intensity } (t) = S(t)/T(t);$$

wherein S(t) is scattered light intensity measured at time point t, and T(t) is transmitted light intensity measured at time point t.

The scattered light signal varies depending on the amount of light incident on the measurement position. By using the scattered light intensity defined above, the apparent scattered light at the measurement position in the solution can be corrected with the attenuated transmitted light.

The light source used for the measurement of the scattered light intensity (and transmitted light intensity in some cases) is not particularly limited, and lamp, semiconductor laser, LED (light emitting diode) and the like can be used. Semiconductor laser is preferable from the aspect that single wavelength can be irradiated. The radiation light wavelength is not particularly limited and a radiation light in the wavelength range of 530 - 780 nm (e.g., 650 nm) is preferably used. For example, when CRP measurement of a whole blood sample is performed, a radiation light of 600 - 780 nm is preferably used to avoid absorption wavelength of hemoglobin.

The scattering angle used for the measurement of scattered light intensity is not particularly limited. It is preferably 3 - 85 degrees, more preferably 35 - 55 degrees, to reduce an influence of a transmitted light in scattered light measurement and receive a scattered light with sufficient intensity.

A calculation method of the index value is not particularly limited as long as it shows a degree of difference between changing speed of the scattered light intensity in the first period and the changing speed of the scattered light intensity in the second period. Specific examples of the index value include a ratio of the two changing speeds (e.g., as described in Example, the ratio of changing speed of scattered light intensity in the latter half of the reaction to the changing speed of scattered light intensity in the former half of the reaction), or a difference between the two changing speeds and the like.

When the index value is determined as mentioned above, whether a concentration of a test substance is within an appropriate range for concentration measurement using an immunoagglutination reaction is determined by comparing the index value with a predetermined threshold value.

The threshold value can be set using an analytical curve drawn using a plurality of samples containing known concentrations of a test substance and the above-mentioned index value obtained for each of these samples. That is, for example, the concentration at which the prozone phenomenon occurs or the concentration before that can be set as the threshold concentration $C_0$ from the analytical curve drawn using a plurality of samples containing known concentrations of a test substance. Further, the index value $A_0$ at the threshold concentration $C_0$ is determined under the same conditions as the measurement method in the determination method of the present invention, and the obtained index value $A_0$ can be determined as the above-mentioned threshold value. Whether the concentration of the test substance in the specimen sample is in or near the prozone region can be determined according to whether the index value determined for the specimen sample is larger or smaller than the thus-set threshold value $A_0$.

(Sample analysis apparatus)

The present invention also provides a sample analysis apparatus. The sample analysis apparatus of the present invention is an apparatus having a processing part to perform the aforementioned determination method of the present invention. Therefore, all the embodiments and preferred embodiments described above in relation to the determination method of the present invention can also be applied to the sample analysis apparatus of the present invention. Therefore, the aforementioned description can be applied to the sample and the test substance to be analyzed by the apparatus.

The sample analysis apparatus of the present invention contains
a scattered light intensity measuring part for measuring intensity of a scattered light;
an index value determining part for, with respect to an immunoagglutination reaction that occurred in the reaction solution between the test substance and a binding partner thereof, determining an index value relating to a difference between a changing speed of a scattered light intensity in a first period of the reaction and a changing speed of a scattered light intensity in a second period of the reaction, based on a scattered light intensity of the reaction solution in the first period and a scattered light intensity of the reaction solution in the second period; and
a first determining part for determining whether the concentration of the test substance is within a range proper for concentration measurement using an immunoagglutination reaction, by comparing the index value and a predetermined threshold value.

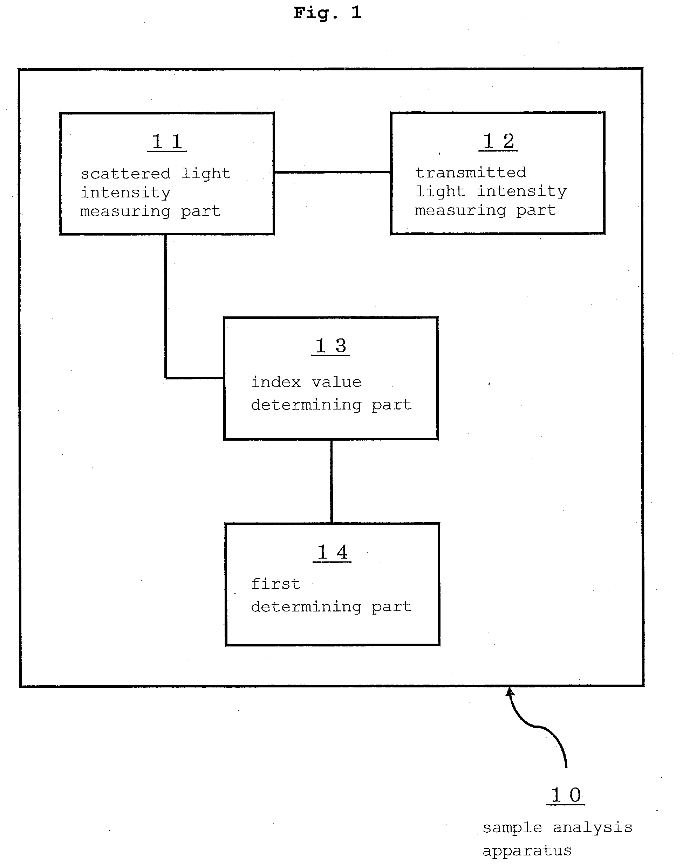

A block diagram of the outline of one embodiment of the sample analysis apparatus of the present invention is shown in Fig. 1.

The sample analysis apparatus 10 in this embodiment is configured including a scattered light intensity measuring part 11, a transmitted light intensity measuring part 12, an index value determining part 13, and the first determining part 14. As described above with respect to the determination method of the present invention, as the scattered light intensity used for determination in the present invention, the measurement value of scattered light intensity corrected with the measurement value of transmitted light intensity can be used. In this example, for this purpose, the apparatus of the present invention includes a transmitted light intensity measuring part 12 in addition to the scattered light intensity measuring part 11, index value determining part 13 and first determining part 14, and these are essential constituent elements in the apparatus of the present invention. In the practice of the present invention, the correction of the scattered light intensity by the transmitted light intensity is not necessarily required, and thus the transmitted light intensity measuring part 12 is not an essential constituent element.

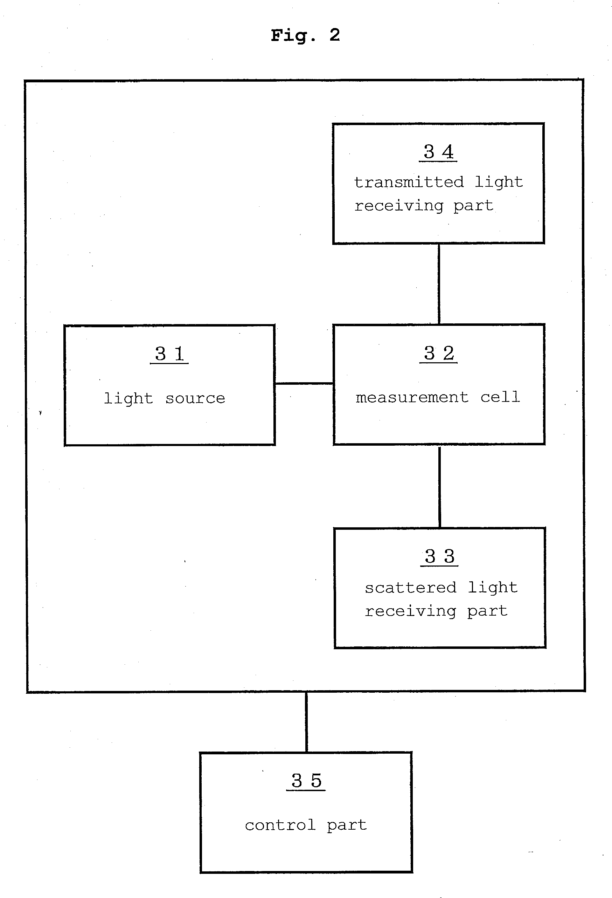

The scattered light intensity measuring part 11 and transmitted light intensity measuring part 12 may be configured as a single optical system as illustrated in the block diagram showing the outline of the example of Fig. 2. The optical system shown in Fig. 2 is configured including a light source 31, a measurement cell 32, a scattered light receiving part 33, a transmitted light receiving part 34 and a control part 35. The light source 31 may be a lamp, a semiconductor laser, LED (light emitting diode) or the like, and a semiconductor laser is preferable since a single wavelength can be irradiated. A sample solution is set in the measurement cell 32, on which a light is irradiated from the light source 31. The scattered light and transmitted light thereof are respectively received by the scattered light receiving part 33 and the transmitted light receiving part 34. These light receiving parts may be configured with light receiving elements such as photo-diode and the like. The analog signals from the scattered light receiving part 33 and transmitted light receiving part 34 are transmitted to the control part 35 and the control part 35 converts them into digital signals. The control part 35 also supplies an electric power to the light source 31, scattered light receiving part 33 and transmitted light receiving part 34 to drive them. The above-mentioned constitution for measuring the scattered light intensity and transmitted light intensity may be similar to the constitution of conventionally-known optical measurement apparatuses. Various parameters such as wavelength of light, scattering angle and the like are as described above for the determination method of the present invention.

The index value determining part 13 and first determining part 14 can be configured as a single control device. In a preferred embodiment, the control device is implemented using a computer.

The index value determining part 13 receives scattered light intensity at each measurement time from, for example, control part 35 of the scattered light intensity measuring part 11, and the data of the progress time, from the start of the reaction, corresponding to the scattered light intensity. The index value determining part 13 determines the aforementioned index value based on these received data, with regard to the determination method of the present invention.

The first determining part 14 determines the appropriateness of the concentration of a test substance in a specimen sample by comparing the index value determined by the index value determining part 13 and the threshold value set in advance in the first determining part 14 by, for example, a user. The method for determining the appropriateness is as described above with respect to the determination method of the present invention.

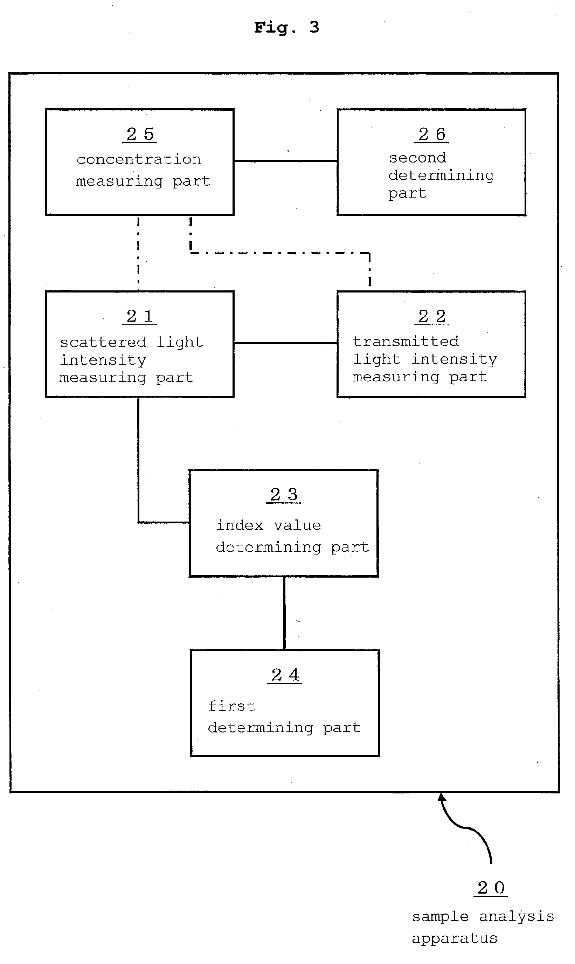

A block diagram showing the outline of other embodiment of the sample analysis apparatus of the present invention is shown in Fig. 3.

The sample analysis apparatus 20 in this example is configured to include concentration measuring part 25 and second determining part 26 in addition to scattered light intensity measuring part 21, transmitted light intensity measuring part 22, index value determining part 23, and the first determining part 24. The scattered light intensity measuring part 21, transmitted light intensity measuring part 22, index value determining part 23, and the first determining part 24 may respectively have constitutions similar to those of the scattered light intensity measuring part 11, transmitted light intensity measuring part 12, index value determining part 13, and first determining part 14, explained with respect to sample analysis apparatus 10 shown in Fig. 1.

The concentration measuring part 25 is a function part for measuring the concentration of a test substance in a specimen sample. The method for measuring the concentration is not particularly limited and, for example, the concentration can be measured by a method known per se and using the measurement value of transmitted light intensity and/or the measurement value of scattered light intensity, and an analytical curve drawn in advance. Therefore, in Fig. 3, concentration measuring part 25 is connected to transmitted light intensity measuring part 22 and scattered light intensity measuring part 21 with a dashed line.

The second determining part 26 is a function part for determining that the concentration of the test substance measured by the concentration measuring part 25 is not within the proper range when the measured concentration is lower than a predetermined threshold value concentration. As described above with respect to the determination method of the present invention, the determination by the second determining part is preferably performed before the determination by the first determining part. The sample analysis apparatus 20 is preferably constituted such that when the second determining part determines that the concentration of the test substance is not within the proper range, it is reported to the user and further processing for determination by the first determining part (i.e., additional measurement of transmitted light and/or scattered light intensity, processing by the index value determining part and the first determining part) is not performed.

The present invention is explained in the following by referring to more concrete Examples; however, the present invention is not limited by the following Examples.

For example, the concentration of C-reactive protein (CRP) in a blood sample can be measured by the following steps. CRP measurement steps

1. Buffer solution is sucked and discharged in a measurement cell.
2. A hemolysis reagent is sucked and discharged in the measurement cell.
3. A specimen is sucked and discharged in the measurement cell.
4. A latex reagent is sucked and discharged in the measurement cell.
5. The solution in the measurement cell is agitated well.
6. An immune reaction occurs and CRP measurement is performed. Change of absorbance is measured for 1 minute per 1 second. The data at that time is put in a calculating part via a signals processing part.
7. When the aforementioned measurement is completed, CRP cell is washed and cleaned with a dilution liquid, whereby all measurements are finished.
8. Based on the data obtained by the measurement, the time change amount of absorbance from 20 seconds to 60 seconds is calculated, and CRP concentration is obtained from the analytical curve previously obtained from serum with known concentration.

(The analytical curve is prepared in advance, in which the time change of absorbance is the horizontal axis and the concentration is the vertical axis.)

By the aforementioned steps and using 10 samples containing CRP at a known concentration, an immunoagglutination reaction with latex conjugated with antibody against CRP was started. Thereafter, the scattered light intensity was measured along with absorbance measurement. In the measurement of the scattered light intensity, signals of scattered light and transmitted light having a wavelength of 650 nm and a scattering angle of 45 degrees were used. The deflection value based on the turbidity (absorbance) and the deflection value based on the scattered light amount were determined for each reaction solution. The deflection value is one embodiment of the index value according to the present invention, and was specifically calculated as follows:

$$\text{Deflection value} = \text{Sp}(40\text{-}59)/\text{Sp}(10\text{-}29)$$

Sp(10-29) is average unit time change amount from 10 seconds to 29 seconds after the start of the reagent reaction.
Sp(40-59) is average unit time change amount from 40 seconds to 59 seconds after the start of the reagent reaction.

Turbidity and scattered light used were determined by the following relationship.

$$\text{Turbidity} = -\log\,(T_t/T_0)$$

$T_0$: transmitted light quantity when detection system is blank

$T_t$: transmitted light quantity at reaction time t

$$\text{Scattered light} = S_t/T_t$$

$S_t$: scattered light quantity at reaction time t

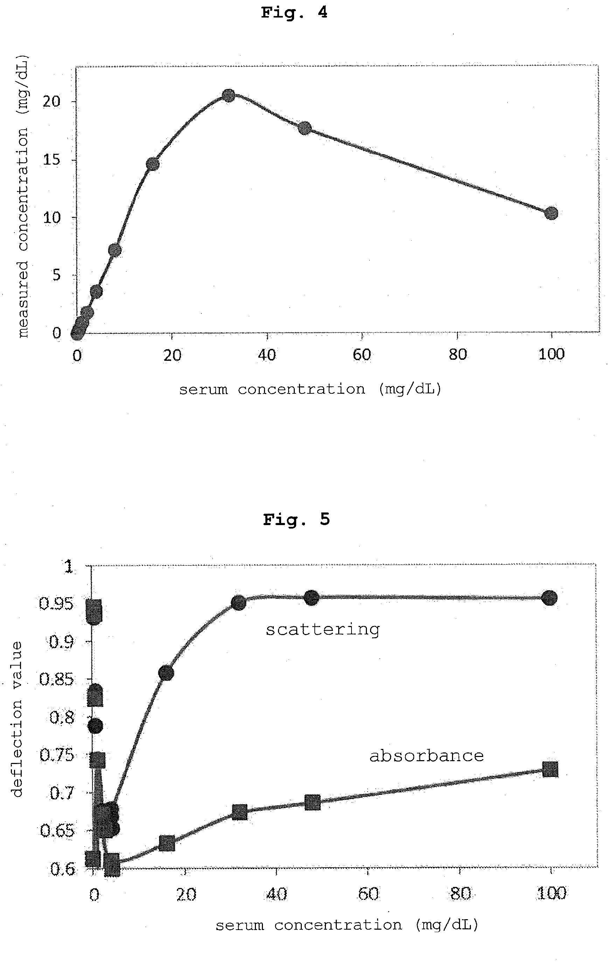

In Fig. 4, the relationship between a serum protein having a known concentration and the measurement values when the measurement was performed using the absorbance is plotted. With the serum concentration of 32 mg/dl as a peak, a serum with higher concentration was observed to show a smaller apparent measurement value. The relationship when the absorbance and deflection value of the scattered light were measured at the same serum concentration is shown in Fig. 5. The deflection value obtained from the absorbance showed large change amounts from 8 to 32 mg/dL, after which increased gradually up to 100 mg/dL.

The deflection value calculated from the scattered light increased rapidly from serum concentration of 8 to 16 mg/dl, after which highly increased up to 32 mg/dL. Thereafter, it showed a constant value irrespective of serum concentration.

For highly accurate determination of prozone, an index having properties to generally show a small value of measurement concentration and a greatly-changed value as it approaches prozone is preferable. In consideration of the influence of the measurement variation, an index having a smaller change width results in a determination at a lower concentration than an index having a larger change width. When the numerical value change amount from 16 mg/dL to 32 mg/dL is $\Delta R_L$ for absorbance and $\Delta R_S$ for scattered light in Fig. 5, the change amount of the numerical value of the scattered light is larger by 1.8 times than that of the numerical value of the absorbance. As this value is larger, the influence of measurement variation is smaller, and more accurate prozone determination is possible. When the threshold value of prozone determination is $D_T$ for absorbance and $D_S$ for scattered light, it is found that a larger allowance of change amount relative to the threshold value is produced by $D_S$ for the scattered light than $D_T$ for the absorbance, and the influence of measurement variation is improved more.

This application is based on a patent application No. 2016-243796 filed in Japan (filing date: December 15, 2016), the contents of which are incorporated in full herein.

[Explanation of Symbols]

| | |
|---|---|
| 10,20 | sample analysis apparatus |
| 11,21 | scattered light intensity measuring part |
| 12,22 | transmitted light intensity measuring part |
| 13,23 | index value determining part |
| 14,24 | first determining part |
| 25 | concentration determining part |
| 26 | second determining part |
| 31 | light source |
| 32 | measurement cell |
| 33 | scattered light receiving part |
| 34 | transmitted light receiving part |
| 35 | control part |

**Claims**

1. A method for determining whether a concentration of a test substance in a reaction solution is within a range proper for concentration measurement using an immunoagglutination reaction, the method comprising:

   a step of, with respect to an immunoagglutination reaction that occurred in the reaction solution between the test substance and a binding partner thereof, determining an index value relating to a difference between a changing speed of a scattered light intensity in a first period of the reaction and a changing speed of a scattered light intensity in a second period of the reaction, based on a scattered light intensity of the reaction solution in the first period and a scattered light intensity of the reaction solution in the second period; and

a step of determining whether the concentration of the test substance is within a proper range by comparing the index value and a predetermined threshold value.

2. The method according to claim 1, wherein the index value is a ratio of the changing speed of the scattered light intensity in the first period and the changing speed of the scattered light intensity in the second period.

3. The method according to claim 1 or 2, wherein a ratio of the measurement value of the scattered light intensity and the measurement value of the transmitted light intensity is used as the scattered light intensity.

4. The method according to any one of claims 1 to 3, wherein the reaction solution is derived from a wet biological sample comprising the test substance.

5. The method according to claim 4, wherein the wet biological sample is selected from the group consisting of blood, saliva, urine, sweat, lymph fluid, and expectoration.

6. The method according to any one of claims 1 to 5, wherein the test substance is selected from the group consisting of protein, peptide, lipid, nucleic acid, polysaccharides, saccharides, and antibody.

7. The method according to any one of claims 1 to 6, wherein the reaction solution is prepared by mixing a specimen sample containing the test substance and a suspension containing insoluble carrier particles carrying the binding partner.

8. The method according to claim 7, wherein the insoluble carrier particle comprises one or more selected from the group consisting of latex, metal colloid, silica, carbon and magnetic material particles.

9. The method according to any one of claims 1 to 8, wherein the concentration in the proper range is a concentration which is neither a concentration causing a prozone phenomenon nor a concentration around same.

10. The method according to any one of claims 1 to 9, further comprising a step of measuring the concentration of the test substance, and determining that the concentration of the test substance is not within the proper range when the measured concentration is lower than a predetermined threshold value concentration.

11. A sample analysis apparatus comprising:

a scattered light intensity measuring part for measuring intensity of a scattered light;
an index value determining part for, with respect to an immunoagglutination reaction that occurred in the reaction solution between the test substance and a binding partner thereof, determining an index value relating to a difference between a changing speed of a scattered light intensity in a first period of the reaction and a changing speed of a scattered light intensity in a second period of the reaction, based on a scattered light intensity of the reaction solution in the first period and a scattered light intensity of the reaction solution in the second period; and
a first determining part for determining whether the concentration of the test substance is within a range proper for concentration measurement using an immunoagglutination reaction, by comparing the index value and a predetermined threshold value.

12. The sample analysis apparatus according to claim 11, wherein the index value is a ratio of the changing speed of the scattered light intensity in the first period and the changing speed of the scattered light intensity in the second period.

13. The sample analysis apparatus according to claim 11 or 12, further comprising a transmitted light intensity measuring part for measuring a transmitted light intensity, wherein a ratio of the measurement value of the scattered light intensity and the measurement value of the transmitted light intensity is used as the scattered light intensity.

14. The sample analysis apparatus according to any one of claims 11 to 13, which is used for analyzing a wet biological sample comprising the test substance.

15. The sample analysis apparatus according to claim 14, wherein the wet biological sample is selected from the group consisting of blood, saliva, urine, sweat, lymph fluid, and expectoration.

16. The sample analysis apparatus according to any one of claims 11 to 15, wherein the first determining part determines whether the concentration of the test substance is a concentration which is neither a concentration causing a prozone phenomenon nor a concentration in the vicinity thereof.

17. The sample analysis apparatus according to any one of claims 11 to 16, further comprising a concentration measuring part for measuring the concentration of the test substance.

18. The sample analysis apparatus according to claim 17, further comprising a second determining part for determining that the concentration of the test substance is not within the proper range when the measured concentration of the test substance is lower than a predetermined threshold value concentration.

**Fig. 1**

```
┌─────────────────────────────────────────────────────────────────┐
│                                                                   │
│   ┌─────────────────────┐        ┌─────────────────────┐          │
│   │        1 1          │        │        1 2          │          │
│   │  scattered light    │────────│  transmitted        │          │
│   │  intensity          │        │  light intensity    │          │
│   │  measuring part     │        │  measuring part     │          │
│   └─────────────────────┘        └─────────────────────┘          │
│              │                                                     │
│              │       ┌─────────────────────┐                      │
│              │       │        1 3          │                      │
│              │       │                     │                      │
│              └───────│  index value        │                      │
│                      │  determining part   │                      │
│                      └─────────────────────┘                      │
│                                 │                                  │
│                      ┌─────────────────────┐                      │
│                      │        1 4          │                      │
│                      │  first              │                      │
│                      │  determining part   │                      │
│                      └─────────────────────┘                      │
│                                                                   │
└─────────────────────────────────────────────────────────────────┘
```

1 0

sample analysis
apparatus

**Fig. 2**

## Fig. 3

## Fig. 4

## Fig. 5

## INTERNATIONAL SEARCH REPORT

| International application No. |
| --- |
| PCT/JP2017/032822 |

| A. CLASSIFICATION OF SUBJECT MATTER |
| --- |
| Int.Cl. G01N33/543(2006.01)i, G01N21/49(2006.01)i, G01N21/75(2006.01)i, G01N21/82(2006.01)i |

According to International Patent Classification (IPC) or to both national classification and IPC

| B. FIELDS SEARCHED |
| --- |
| Minimum documentation searched (classification system followed by classification symbols) |
| Int.Cl. G01N33/543, G01N21/49, G01N21/75, G01N21/82 |

| Documentation searched other than minimum documentation to the extent that such documents are included in the fields searched |
| --- |
| Published examined utility model applications of Japan 1922–1996 |
| Published unexamined utility model applications of Japan 1971–2017 |
| Registered utility model specifications of Japan 1996–2017 |
| Published registered utility model applications of Japan 1994–2017 |

Electronic data base consulted during the international search (name of data base and, where practicable, search terms used)

### C. DOCUMENTS CONSIDERED TO BE RELEVANT

| Category* | Citation of document, with indication, where appropriate, of the relevant passages | Relevant to claim No. |
| --- | --- | --- |
| A | JP 6-17914 B2 (MITSUBISHI KASEI CORP.) 09 March 1994, claims & JP 59-171863 A | 1–18 |
| A | JP 2508115 B2 (SHIMADZU CORP.) 19 June 1996, claims & JP 64-29743 A | 1–18 |
| A | JP 2946850 B2 (SHIMADZU CORP.) 06 September 1999, claims & JP 5-10953 A | 1–18 |

☒ Further documents are listed in the continuation of Box C.  ☐ See patent family annex.

| * Special categories of cited documents: | "T" later document published after the international filing date or priority date and not in conflict with the application but cited to understand the principle or theory underlying the invention |
| --- | --- |
| "A" document defining the general state of the art which is not considered to be of particular relevance | |
| "E" earlier application or patent but published on or after the international filing date | "X" document of particular relevance; the claimed invention cannot be considered novel or cannot be considered to involve an inventive step when the document is taken alone |
| "L" document which may throw doubts on priority claim(s) or which is cited to establish the publication date of another citation or other special reason (as specified) | "Y" document of particular relevance; the claimed invention cannot be considered to involve an inventive step when the document is combined with one or more other such documents, such combination being obvious to a person skilled in the art |
| "O" document referring to an oral disclosure, use, exhibition or other means | |
| "P" document published prior to the international filing date but later than the priority date claimed | "&" document member of the same patent family |

| Date of the actual completion of the international search | Date of mailing of the international search report |
| --- | --- |
| 17 November 2017 (17.11.2017) | 28 November 2017 (28.11.2017) |

| Name and mailing address of the ISA/ Japan Patent Office 3-4-3, Kasumigaseki, Chiyoda-ku, Tokyo 100-8915, Japan | Authorized officer |
| --- | --- |
| | Telephone No. |

Form PCT/ISA/210 (second sheet) (January 2015)

**INTERNATIONAL SEARCH REPORT**

International application No.

PCT/JP2017/032822

C (Continuation).   DOCUMENTS CONSIDERED TO BE RELEVANT

| Category* | Citation of document, with indication, where appropriate, of the relevant passages | Relevant to claim No. |
|---|---|---|
| A | JP 5-93725 A (SHIMADZU CORP.) 16 April 1993, claims (Family: none) | 1-18 |
| A | JP 7-83929 A (TOKUYAMA CORPORATION) 31 March 1995, abstract (Family: none) | 1-18 |
| A | JP 60-196669 A (HITACHI, LTD.) 05 October 1985 (Family: none) | 1-18 |
| A | JP 10-111248 A (DADE BEHRING MARBURG GMBH) 28 April 1998 & US 6044330 A & US 6317702 B1 & EP 833153 A2 | 1-18 |
| A | JP 2909140 B2 (A&T CORPORATION) 23 June 1999 & JP 4-5568 A | 1-18 |
| A | US 2012/0094394 A1 (BECKMAN COULIER, INC.) 19 April 2012 & WO 2010/118861 A1 & EP 2241886 A1 & CN 102395885 A | 1-18 |

Form PCT/ISA/210 (continuation of second sheet) (January 2015)

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2014192963 A1
- JP 2016243796 A